(19) Europäisches Patentamt
European Patent Office
Office européen des brevets

(11) Veröffentlichungsnummer: **0 115 574**
**B1**

# (12) EUROPÄISCHE PATENTSCHRIFT

(45) Veröffentlichungstag der Patentschrift: **28.01.87**

(51) Int. Cl.⁴: **C 08 B 37/08**, A 61 K 7/06

(21) Anmeldenummer: **83110950.9**

(22) Anmeldetag: **03.11.83**

(54) **Kosmetisches Mittel auf der Basis von quaternären Chitosanderivaten, neue quaternäre Chitosanderivate sowie Verfahren zu ihrer Herstellung.**

(30) Priorität: **10.12.82 DE 3245784**

(43) Veröffentlichungstag der Anmeldung: **15.08.84 Patentblatt 84/33**

(45) Bekanntmachung des Hinweises auf die Patenterteilung: **28.01.87 Patentblatt 87/5**

(84) Benannte Vertragsstaaten: **AT DE FR GB IT NL SE**

(56) Entgegenhaltungen:
**EP-A-0 002 506**
**EP-A-0 065 491**
**US-A-2 063 671**
**US-A-3 876 760**

(73) Patentinhaber: **Wella Aktiengesellschaft, Berliner Allee 65, D-6100 Darmstadt (DE)**

(72) Erfinder: **Lang, Günther, Dr., Auf der Roten Erde 10 B, D-6107 Reinheim 5 (DE)**
Erfinder: **Wendel, Harald, Grabengasse 3, D-6105 Ober-Ramstadt (DE)**
Erfinder: **Konrad, Eugen, Mecklenburger Strasse 101, D-6100 Darmstadt (DE)**

**EP 0 115 574 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft kosmetische Mittel zur Behandlung von Haaren oder der Haut mit einem Gehalt an neuen makromolekularen quaternären, vom Chitosan abgeleiteten Verbindungen in einer geeigneten Kosmetikgrundlage.

Die Erfindung betrifft weiterhin die neuen quaternären Chitosanderivate sowie Verfahren zu ihrer Herstellung.

Es ist bereits bekannt, in kosmetischen Mitteln, insbesondere für die Behandlung von Haaren, kationaktive Polymere, insbesondere Polymere, die quaternäre Ammoniumgruppen aufweisen, als Konditionierungsmittel einzusetzen. Auf Grund einer Wechselwirkung zwischen ihren Ammoniumgruppen und den anionischen Gruppen des Haares besitzen die kationaktiven Polymere eine grosse Affinität zur Keratinfaser.

Es wurde festgestellt, dass der Einsatz derartiger kationaktiver Polymere in solchen kosmetischen Mitteln zahlreiche Vorteile ergibt; die Entwirrung der Haare sowie deren Behandlung wird erleichtert, und weiterhin wird dem Haar Sprungkraft und Glanzwirkung verliehen. Durch die Affinität zum Keratin neigen diese Polymere jedoch bei wiederholter Anwendung zur Ansammlung auf den Haaren, so dass diese schwerer werden, was im Endeffekt unerwünscht ist.

Weiterhin ergeben sich bei synthetischen Polymeren Probleme wegen der physiologischen Wirkung von eventuell vorhandenen Monomerspuren, die nur schwer aus dem Polymer entfernbar sind.

Man hat bereits versucht, diese vorerwähnten Nachteile dadurch zu beheben, dass wasserlösliche Salze des Chitosans, eines durch Entacetylierung von Chitin herstellbaren Polyglucosamins, in derartigen kosmetischen Mitteln Anwendung finden. In diesem Zusammenhang wird Bezug genommen auf die eigene europäische Patentschrift 0 002 506 sowie die eigene deutsche Patentschrift 26 27 419.

In gleicher Weise wie bei der Mehrzahl der kationaktiven Polymere mit quaternären Gruppierungen ergibt Chitosan ebenfalls häufig den Nachteil, dass es mit den anionaktiven oberflächenaktiven Agentien, die üblicherweise in kosmetischen Mitteln zur Behandlung von Haaren, insbesondere in Shampoos, Anwendung finden, wenig verträglich ist. Es ist daher notwendig, das Chitosan in separaten Behandlungen, nämlich vor und/oder nach der Shampoonierung zur Einwirkung zu bringen.

Das Chitosan erweist sich weiterhin in neutralem und alkalischem Medium als praktisch unlöslich, so dass seine Anwendung beispielsweise in alkalischen Dauerwellmitteln oder Haarfärbemitteln nicht möglich ist. Aufgabe der Erfindung ist es, diese Nachteile zu beseitigen.

Bei Fortführung der Untersuchungen mit Chitosan und den davon abgeleiteten Verbindungen wurde nunmehr gefunden, dass bestimmte quaternäre Chirosanderivate die vorstehend aufgeführten Nachteile nicht aufweisen.

Mit diesen quaternären Chitosanderivaten lassen sich somit kosmetische Mittel zur Behandlung von Haaren oder der Haut herstellen, die sich durch überraschend vorteilhafte Eigenschaften auszeichnen und die dadurch gekennzeichnet sind, dass sie in einer geeigneten Kosmetikgrundlage eine quaternäre makromolekulare, vom Chitosan abgeleitete polymere Verbindung der allgemeinen Formel I

$$HO\left[\ C_6H_{11-m}NO_4(R^1)_m(R^2)_n(R^3)_q\ \right]_pH, \qquad (I)$$

wobei m jeden beliebigen Zahlenwert von 0 bis 0,5 bedeutet, n jeden beliebigen Zahlenwert von 0 bis 6 bedeutet, q ein beliebiger Zahlenwert von 0,005 bis 3,0 ist, p eine ganze Zahl von 10 bis 50 000 bedeutet, $R^1$ ein Acetyl-Rest ist, $R^2$ einen zweiwertigen Rest $-CH_2CH(OH)-CH_2-O-$, $-CH_2-\underset{\displaystyle CH_2-OH}{\underset{|}{C}}H-O-$ oder $-\underset{\displaystyle CH_2-O-}{\underset{|}{C}}H-CH_2-OH$ darstellt und

$R^3$ ein zweiwertiger Rest

$$-CH_2-\underset{\displaystyle (R^4)_3N^+X^-}{\underset{|}{\underset{\displaystyle CH_2}{\underset{|}{C}}}}H-O- \qquad \text{oder} \qquad -\underset{\displaystyle (R^4)_3N^+X^-}{\underset{|}{\underset{\displaystyle CH_2}{\underset{|}{C}}}}H-CH_2-O-$$

mit $R^4 = C_1$- bis $C_4$-Alkyl und X = Cl, Br, J oder $CH_3SO_4$ ist, enthalten.

Die erfindungsgemässen, quaternäre Chitosanderivate der Formel I enthaltenden Mittel eignen sich ganz allgemein zur Behandlung der Haut und/oder der Haare. Sie können beispielsweise vorliegen als Haar- und/oder Körperwaschmittel, Tönungsshampoos, Frisiercremes, Frisierlotionen, Fönlotionen, Mittel zur Festigung der Frisur, Waschlotionen, Haarkuren, Mittel gegen Kopfschuppen, Mittel zur permanenten Haarverformung, Mittel zur Färbung oder Entfärbung von Haaren, Mittel zum Auftragen vor oder nach der Haarfärbung und als kosmetische Mittel zur Pflege, zum Schutz oder zur Reinigung der Haut wie Gesichtswässer, Rasierwässer, Feuchthaltecremes, Coldcremes, Körperlotionen, Sonnenschutzmittel oder auch Make-up-Präparate wie Schminkcremes und Rouges.

Der Gehalt der erfindungsgemässen kosmetischen Mittel an den neuen Chitosanderivaten der Formel I liegt zweckmässig bei 0,05 bis 10 Gew.-%, vorzugsweise bei 0,05 bis 3,0 Gew.-%.

Die kosmetischen Mittel gemäss der vorliegenden Erfindung können zusätzlich zu dem neuen Chitosanderivat der Formel I zur Herstellung einer Kosmetikgrundlage alle diejenigen Bestandteile enthalten, die in Haar- und Hautbehandlungsmitteln üblicherweise eingesetzt werden, insbesondere anionische, kationische, amphotere, zwitterionische oder nichtionische oberflächenaktive Tenside, Schaumsynergisten, Stabilisatoren, Sequestrierungsmittel, Pigmente, Verdicker, Emulgatoren, Pufferstoffe, Konservierungsmittel, Farbstoffe, Parfümöle, bekannte kosmetische Polymere wie anionische, nichtionische, kationische oder amphotere Polymere, Naturstoffe, kosmetische Öle, Fettalkohole, Wachse, Schaumstabilisatoren, Wirkstoffe gegen Kopfschuppen, Reduktionsmittel und Treibgase.

Die erfindungsgemässen kosmetischen Mittel weisen in bevorzugter Weise einen pH-Wert von 2 bis 11 auf und können in Form wässriger, alkoholischer oder wässrig-alkoholischer Zubereitungen, z.B. mit einem Alkohol mit 1 bis 4 Kohlenstoffatomen, als Lösungen, als Cremes, als Gele, als Dispersionen oder als Emulsionen vorliegen. Ebenfalls ist es möglich, diese Mittel mit Hilfe eines Zerstäubers bzw. anderer geeigneter Sprühvorrichtungen oder im Gemisch mit üblichen Treibgasen als Aerosolspray aus einem Druckbehälter zu versprühen.

Wenn es sich bei den erfindungsgemässen kosmetischen Mitteln um Mittel zur Festigung der Frisur, wie flüssige Haarfestiger oder Haarsprays, handelt, dann liegen sie üblicherweise als wässrige oder wässrig-alkoholische Lösungen vor, die durch einen Gehalt an quaternären Chitosanderivaten der vorstehend genannten Formel I gekennzeichnet sind. Hierbei können die quaternären Chitosanderivate selbst als filmbildendes bzw. festigendes Harz eingesetzt werden. Es können jedoch auch zusätzlich andere filmbildende natürliche oder synthetische Polymere in dem erfindungsgemässen Haarfestigungsmittel enthalten sein. Als natürliche Polymere kommen beispielsweise Schellack, Alginate, Gelatine, Pektine und Cellulosederivate in Betracht. Von den synthetischen Polymeren finden z.B. Polyvinylpyrrolidon, Polyvinylacetat, Polyacrylverbindungen, wie Acrylsäure- oder Methacrylsäurepolymerisate, basische Polymerisate von Estern aus Acrylsäure oder Methacrylsäure mit Aminoalkoholen bzw. die Salze oder Quaternisierungsprodukte dieser basischen Polymerisate, Polyacrylnitril sowie Co- oder Terpolymerisate aus derartigen Verbindungen, beispielsweise Polyvinylpyrrolidon-Vinylacetat, Verwendung. Die Mittel weisen dann insbesondere einen pH-Wert zwischen 6 und 8 auf. Solche Mittel zur Festigung der Frisur enthalten üblicherweise filmbildende Polymere in einer Gesamtmenge von etwa 0,05 bis 3,0 Gew.-%. Enthalten die Mittel neben den quaternären Chitosanderivaten der Formel I noch andere filmbildende Polymere, so reduziert sich der Gehalt an quaternären Chitosanderivaten entsprechend.

Als Alkohole kommen insbesondere die für kosmetische Zwecke üblicherweise verwendeten niederen Alkohole wie Ethylalkohol und Isopropylalkohol in Betracht.

Die erfindungsgemässen Mittel zur Festigung der Frisur können weiterhin die üblichen Zusätze wie beispielsweise Parfümöl, Bakterizide oder Fungizide, kämmbarkeitsverbessernde Substanzen usw. enthalten.

Die erfindungsgemässen Mittel zur Festigung der Frisur können gegebenenfalls durch einen Gehalt an kosmetischen Farbstoffen das Haar gleichzeitig färben oder tönen. Derartige Präparate sind u.a. als Farbfestiger oder Tönungsfestiger im Handel bekannt. Sie enthalten zusätzlich übliche für Haarfestiger bekannte Farbstoffe wie beispielsweise aromatische Nitrofarbstoffe (z.B. 1,4-Diamino-2-nitrobenzol), Azofarbstoffe (z.B. C.I. Acid Brown 4), Anthrachinonfarbstoffe (z.B. C.I. Disperse Violet 4) und Triphenylmethanfarbstoffe (z.B. C.I. Basic Violet 1), wobei die Farbstoffe dieser Klassen je nach der Art ihrer Substituenten sauren, nichtionogenen oder basischen Charakter haben können. Ihre Gesamtkonzentration in diesen Präparaten beträgt üblicherweise etwa 0,01 bis 2,0 Gew.-%.

Die erfindungsgemässen Mittel zur Festigung der Frisur weisen bei gleich guter Festigung des Haars gegenüber üblichen Mitteln eine verbesserte Substantivität zum Haar, eine besonders gute Kämmbarkeit und einen guten Griff des Haares im nassen Zustand sowie einen besonders angenehmen Griff des Haars im getrockneten Zustand auf.

Wenn die erfindungsgemässen Mittel Haarwaschmittel darstellen, liegen sie in Form wässriger Lösungen oder Emulsionen vor und enthalten neben dem neuen Chitosanderivat zumindest ein anionisches, kationisches, nichtionisches oder amphoteres Tensid.

In diesem Haarwaschmittel liegt die Konzentration des Tensids im allgemeinen zwischen 3 und 50 Gew.-% und vorzugsweise zwischen 3 und 25 Gew.-%, bezogen auf das Gesamtgewicht des Mittels, wobei der pH-Wert im allgemeinen zwischen 3 und 9 und vorzugsweise zwischen 4 und 7 liegt.

Die erfindungsgemässen Mittel, die in Form von Haarwaschmitteln vorliegen, enthalten im allgemeinen verschiedene Zusatzstoffe, insbesondere Parfüms, Konservierungsstoffe, Verdicker, Schaumstabilisatoren, Puffersubstanzen, kosmetische Harze, Pigmente und Farbstoffe.

Unter den Schaumstabilisatoren können die Fettamide und insbesondere die Mono- oder Diethanolamide von Copra-Fettsäuren, Lauryl- oder Ölsäuremono- oder -diethanolamid, die zweckmässigerweise in Mengen von 1 bis 10 und vorzugsweise 1 bis 3 Gew.-%, bezogen auf das Gesamtgewicht des Mittels Verwendung finden, angeführt werden.

Unter den Verdickern können insbesondere die Acrylpolymere und die Cellulosederivate wie Carboxymethylcellulose, Hydroxypropylmethylcellulose, Hydroxyethylcellulose angeführt werden. Die Verdicker liegen im allgemeinen in einem Anteil von 0,1 bis 5 Gew.-% vor.

Unter den Tensiden oder oberflächenaktiven Agentien, die in Kombination mit den neuen quaternären Chitosanderivaten verwendet werden, können beispielsweise die folgenden genannt werden:

a) die anionischen oberflächenaktiven Agentien, wie beispielsweise die Alkali- oder Erdalkalisalze der Alkanolamine von Alkansulfonaten, Alkylsulfaten und Alkylethersulfaten, die $C_{12}$–$C_{18}$-Alkyl- und insbesondere $C_{12}$–$C_{14}$-Alkyl-Sulfatnatriumsalze oder Triethanolaminsalze, die Natri-

um- oder Triethanolaminsalze von Lauryl- oder Tetradecylethersulfaten, das Dinatriumsalz des Sulfosuccinhalbesters von Alkanolamiden, die Seifen und die Polyethercarbonsäuren;

b) die nichtionischen oberflächenaktiven Agentien, wie beispielsweise oxethylierte Fettalkohole mit 12 bis 18 Kohlenstoffatomen, z.B., mit bis zu 40 Mol Ethylenoxid pro Mol Fettalkohol, oxethylierter Laurin-, Tetradecyl-, Cetyl-, Olein-, Palmitin- und Stearinalkohol, allein oder im Gemisch, die Fettalkohole von oxethyliertem Lanolin oder oxethyliertes Lanolin; Polyglycerylether von gesättigten oder ungesättigten Fettalkoholen und Alkylphenolen mit 8 bis 30 Kohlenstoffatomen im Alkylrest und 1 bis 10 Glyceryleinheiten im Molekül sowie Fettsäurealkanolamide;

c) die kationischen oberflächenaktiven Agentien, wie beispielsweise das Dilauryldimethylammoniumchlorid, die Chloride oder Bromide von Alkyldimethylbenzylammonium, die Chloride oder Bromide von Alkyltrimethylammonium, beispielsweise Cetyltrimethylammoniumchlorid oder -bromid, Tetradecyltrimethylammoniumchlorid oder -bromid, die Alkyldimethylhydroxyethylammoniumchloride oder -bromide, die Dialkyldimethylammoniumchloride oder -bromide, Alkylpyridinumsalze, beispielsweise Cetylpyridinumchlorid, die Alkylamidethyltrimethylammoniumethersulfate, Imidazolinderivate, Verbindungen mit kationischem Charakter wie Aminoxide, beispielsweise Alkyldimethylaminoxide oder Alkylaminoethyldimethylaminoxide;

d) die amphoteren oder zwitterionischen oberflächenaktiven Agentien wie beispielsweise die Carboxylderivate von Imidazol, die N-Alkylbetaine, die N-Alkylsulfobetaine, die N-Alkylaminobetaine, die N-Alkylaminopropionate, die Alkyldimethylammoniumacetate bzw. die $C_{12}$–$C_{18}$-Alkyldimethylcarboxymethylammoniumsalze.

Die erfindungsgemässen kosmetischen Mittel können auch Cremes oder Lotionen zur Verwendung als Haarkur oder Hautpflegemittel darstellen. Sie liegen dann meist in Form von Öl-in-Wasser- oder Wasser-in-Öl-Emulsionen oder Suspensionen vor und enthalten zusätzlich zu den neuen Chitosanderivaten der Formel I kationische, nichtionogene, amphotere oder anionische Emulgatoren sowie als Bestandteil der Ölphase z.B. Fettalkohole, Fettsäureester- oder -amide, weiterhin Parfümöle, Vaseline, Wollfettalkohol oder feste oder flüssige Paraffine.

Wenn die erfindungsgemässen Mittel Haartönungs- oder Haarfärbemittel darstellen, so liegen sie ebenfalls bevorzugt in Form von Cremes oder Lotionen vor und enthalten zusätzlich übliche Haarfarbstoffe aus der Gruppe der aromatischen Nitrofarbstoffe, Azofarbstoffe, Anthrachinonfarbstoffe, Triphenylmethanfarbstoffe oder auch Oxidationsfarbstoffe, beispielsweise aus der Gruppe der aromatischen Diamide bzw. Aminophenole. Weiterhin können diese Mittel gegebenenfalls Alkalisierungsmittel, Antioxidantien sowie weitere für solche Mittel übliche kosmetische Zusätze und Hilfsstoffe enthalten.

Die erfindungsgemässen Mittel können auch Dauerverformungsmittel oder Fixiermittel für Haare darstellen. Sie enthalten dann zusätzlich zu den genannten Chitosanderivaten der Formel I Reduktionsmittel, wie Thioglykolsäure, Thiomilchsäure, Ammoniumsulfit bzw. Oxidationsmittel, wie Wasserstoffperoxid oder Natriumbromat sowie gegebenenfalls Alkalisierungsagentien bzw. Peroxidstabilisatoren, z.B. Phosphorsäure, und andere kosmetische Hilfsstoffe und Zusatzstoffe wie beispielsweise Parfümöle, Riechstoffe, Pflegestoffe und Farbstoffe.

Wie bereits erwähnt wurde, können die erfindungsgemässen kosmetischen Mittel auch zur Behandlung der Haut verwendet werden.

In der Tat erleichtern diese kosmetischen Mittel die Befeuchtung der Haut und verhindern das Austrocknen. Diese Mittel verleihen der Haut weiterhin eine hervorragende Weichheit im Griff.

Die erfindungsgemässen kosmetischen Mittel liegen hierzu vorzugsweise in Form von Cremes, Gelen, Emulsionen oder wässrigen, alkoholischen oder wässrig-alkoholischen Lösungen vor, die das Chitosanderivat der Formel I in einer Konzentration von 0,1 bis 10 Gew.-% und vorzugsweise von 0,2 bis 6 Gew.-% enthalten.

Die im allgemeinen in diesen Kosmetikzubereitungen enthaltenen Hilfsstoffe sind beispielsweise Duftstoffe, Farbstoffe, Konservierungsmittel, Verdickungsmittel, Sequestriermittel, Emulgiermittel, Sonnenschutzfilter etc.

Diese Zubereitungen für die Hautpflege liegen insbesondere in Form von Cremes oder Lotionen zur Pflege der Hände oder des Gesichts oder in Form von Sonnenschutzcremes, gefärbten Cremes, Abschminkmilchprodukten, Schaumbad- und Duschbad-Präparaten oder auch in Form von Deodorierzubereitungen vor.

Diese Zubereitungen werden unter Anwendung klassischer Verfahrensweisen hergestellt.

Beispielsweise kann man zur Bildung einer Creme eine wässrige Phase, die das erfindungsgemässe Chitosanderivat und gegebenenfalls andere Bestandteile oder Hilfsstoffe gelöst enthält, und eine ölige Phase emulgieren.

Für die ölige Phase kann man verschiedenartige Verbindungen verwenden, beispielsweise Paraffinöl, Vaselinöl, Süssmandelöl, Avocadoöl, Olivenöl, Fettsäureester, wie Glycerylmonostearat, Äthylpalmitat oder Isoproylpalmitat oder Alkylmyristate, wie Propylmyristat, Butylmyristat oder Cetylmyristat. Man kann sie auch mit Fettsäurealkoholen, wie Cetylalkohol oder Wachsen, beispielsweise Bienenwachs, versetzen.

Die Chitosanderivate der Formel I können in den Kosmetikzubereitungen für die Hautpflege entweder als Hilfsstoff oder als Hauptwirkstoff enthalten sein.

Die in den erfindungsgemässen kosmetischen Mitteln enthaltenen neuen Chitosan-Derivate leiten sich von Chitosan ab, einem Material, das durch Entacetylierung von Chitin, einem natürlich vorkommenden Acetylglucosamin, erhalten wird.

Das Chitosan ist im neutralen und alkalischen Medium unlöslich, es bildet jedoch auf Grund

seiner chemischen Natur im sauren Medium mit organischen und anorganischen Säuren Salze, die beispielsweise in der Papier- und Textilindustrie als Additive Verwendung finden sowie weiterhin als Koagulanzien für Suspensionen, als Chelatbildner für Schwermetallionen sowie in der Medizin und in der Kosmetik benutzt werden (siehe in diesem Zusammenhang die Veröffentlichung von Muzarelli: «Chitin», Pergamon Press, 1977).

Es sind auch bereits einige wasserlösliche Chitosanderivate bekannt, beispielsweise Carboxymethylchitosan, Sulfoethylchitosan (siehe Nudğa, Plisko und Darnilov, Zhur. Prikl. Khim. 47, 872–875). Diese wasserlöslichen Chitosanderivate sind jedoch in ihrem ionischen Charakter verändert oder aber physiologisch bedenklich (Epichlorhydrinchitosan, Veröffentlichung von Noguchi, Arato und Komai; Kogyo Kagaku Zasshi 72, 769–799 und japanische Patentanmeldung Nr. 43–39 322, H. Haga).

Diese vorerwähnten polymeren Verbindungen erfordern ausserdem für ihre technische Herstellung relativ aufwendige Verfahren.

Es wurde nunmehr gefunden, dass sich durch Umsetzung von Chitosan mit einem Glycidyltrialkylammoniumhalogenid (Oxyranmethanamminium-N,N,N-trialkylhalogenid) der Formel

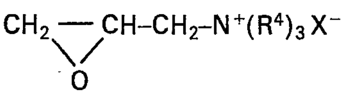

($R^4$ = $C_1$- bis $C_4$-Alkyl; X = Cl, Br, J oder $CH_3SO_4$) sowie gegebenenfalls zusätzlich mit Glycidol (1,2-Epoxypropanol-3) in einfacher Weise quaternäre Chitosanderivate mit hoher Substantivität, unter anderem zu Haarkeratin, herstellen lassen.

Die neuen quaternären makromolekularen, vom Chitosan abgeleiteten polymeren Verbindungen sind gekennzeichnet durch die allgemeine Formel I

$$HO\left[\ C_6H_{11-m}NO_4(R^1)_m(R^2)_n(R^3)_q\ \right]_pH, \qquad (I)$$

wobei m jeden beliebigen Zahlenwert von 0 bis 0,5 bedeutet, n jeden beliebigen Zahlenwert von 0 bis 6 bedeutet, q ein beliebiger Zahlenwert von 0,005 bis 3,0 ist, p eine ganze Zahl von 10 bis 50 000 bedeutet, $R^1$ ein Acetyl-Rest ist, $R^2$ einen zweiwertigen Rest

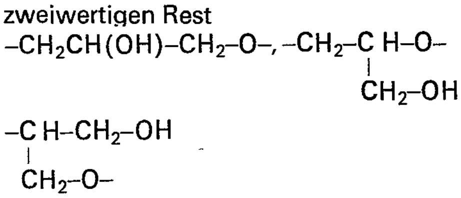

oder

darstellt und $R^3$ ein zweiwertiger Rest

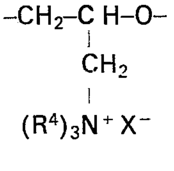

oder

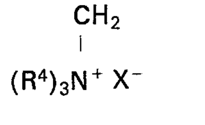

mit $R^4$ = $C_1$- bis $C_4$-Alkyl und X = Cl, Br, J oder $CH_3SO_4$ ist, wobei der Ausdruck in eckigen Klammern sich wiederholende substituierte Glucosamin-Monomereinheiten darstellen soll.

Die neuen, quaternären Stickstoff enthaltenden Chitosanderivate werden erfindungsgemäss hergestellt, indem man ein Chitosan, bestehend aus zu 50–100% entacetyliertem Chitin, in Gegenwart eines Lösungsmittels mit einem Glycidyltrialkylammoniumhalogenid sowie gegebenenfalls zusätzlich mit Glycidol in geeignetem Verhältnis gleichzeitig oder in beliebiger Reihenfolge zur Umsetzung bringt. Falls die Umsetzung in Gegenwart von Glycidol durchgeführt wird, kann auf ein Lösungsmittel verzichtet werden, wobei dann ein Überschuss an Glycidol als Lösungsmittel dienen kann.

Geeignete Glycidyltrialkylammoniumhalogenide sind zum Beispiel Glycidyltrimethylammoniumchlorid und Glycidyltriethylammoniumchlorid. Die den quaternären Stickstoff enthaltenden Epoxide können aber auch in situ durch Umsetzung der entsprechenden Chlorverbindung mit basischen Katalysatoren (beispielsweise Umsetzung von 1-Chlor-3-trimethylammonium-propanol-2-chlorid mit Natronlauge) vor oder während der Umsetzung mit dem Chitosan erzeugt werden.

Die Umsetzung des Chitosans mit Glycidol und dem quaternären Epoxyd kann in einfacher Weise durch gleichzeitige Umsetzung mit beiden Komponenten erfolgen, es ist jedoch auch möglich, die Umsetzung in 2 Stufen durchzuführen. So kann zunächst das Chitosan mit dem Glycidol und das erhaltene Produkt mit dem quaternären Epoxyd oder einem Gemisch aus Glycidol und dem quaternären Epoxyd umgesetzt oder das Ausgangschitosan nur mit dem quaternären Epoxyd und gegebenenfalls anschliessend mit Glycidol umgesetzt werden.

Hierzu wird zweckmässig das Chitosan in feingepulverter Form eingesetzt. Die Umsetzung selbst kann bei Temperaturen zwischen 10 und 100 °C, vorzugsweise zwischen 50 und 80 °C, erfolgen, und diese Temperatur wird zweckmässig unter Rühren 2 bis 100 h beibehalten. Die Reaktion kann mit oder ohne saure oder basische Katalysatoren in Gegenwart von Lösungsmitteln oder, bei Verwendung eines Überschusses an Glycidol, auch ohne ein besonderes Lösungsmittel erfolgen.

Vorzugsweise wird die Reaktion jedoch in Gegenwart von Wasser durchgeführt. Es hat sich dabei als vorteilhaft erwiesen, ohne zusätzliche Katalysatoren zu arbeiten, wobei das Verhältnis von Chitosan zu Wasser zwischen 1:0,05 und 1:100 und das Verhältnis von Chitosan zur Gesamtmenge aus dem Glycidol und dem Glycidyltrialkylammoniumhalogenid (bezogen auf die Mole von substituierbaren Aminogruppen am Chitosan) zwischen 1:0,5 und 1:30, vorzugsweise zwischen 1:1 und 1:10, liegt. Falls das Chitosan auch mit Glycidol zur Umsetzung gebracht wird, soll das Verhältnis zwischen dem Glycidol und dem Glycidyltrialkylammoniumhalogenid, je nach gewünschtem Substitutionsgrad, zweckmässigerweise zwischen 1:100 und 100:1, vorzugsweise zwischen 1:10 und 10:1, liegen.

Obwohl die Durchführung der Reaktion in Gegenwart von Wasser bevorzugt ist, kann sie auch unter Verwendung anderer Lösungsmittel, in denen mindestens eines der Reaktionsprodukte löslich ist, durchgeführt werden. Beispiele für solche Lösungsmittel sind Alkohole wie Ethanol, Methanol, Glykol und Glycerin sowie Ketone, beispielsweise Methylethylketon und Aceton.

Gemäss einer anderen Ausführungsform des erfindungsgemässen Verfahrens zur Herstellung der Chitosanderivate der Formel I werden bei der Umsetzung zusätzlich organische oder anorganische Säuren oder Basen als Katalysatoren zugesetzt.

Für einen Einsatz als Katalysatoren geeignete Säuren sind zum Beispiel Salzsäure, Milchsäure und Ameisensäure. Beispiele für geeignete Basen sind Trialkylamine wie beispielsweise Trimethylamin, Triethylamin oder Trialkylolamine sowie Alkalihydroxide und Erdalkalihydroxide. Es ist grundsätzlich auch möglich, von wasserlöslichen Salzen des Chitosans, beispielsweise Chitosanlaktat, Chitosanacetat, Chitosanhydrochlorid usw. auszugehen. Hierbei kann jedoch als Nebenprodukt eine grössere Menge der Glycerinester der verwendeten Säure entstehen, so dass die Reinigung der entstandenen Reaktionsprodukte erschwert wird.

Die Aufarbeitung der Reaktionsgemische kann beispielsweise in der Weise erfolgen, dass man das Lösungsmittel und gegebenenfalls überschüssiges Glycidol im Vakuum aus der Reaktionsmischung abdestilliert.

Bei der Herstellung von wasserlöslichen quaternären Chitosanderivaten kann das Reaktionsprodukt in bevorzugter Weise in einem Überschuss an Wasser gelöst und durch Filtration oder Zentrifugieren vom nicht-löslichen Reaktionsrückstand abgetrennt werden. Als weitere Schritte zur Reinigung der Reaktionsprodukte können die wässrigen Lösungen dialysiert werden und/oder gegebenenfalls nach Einengen der wässrigen Lösungen durch Ausfällen in Aceton, Alkoholen oder anderen organischen Lösungsmitteln isoliert werden.

Gemäss einer besonders vorteilhaften Ausführungsform des erfindungsgemässen Herstellungsverfahrens wird als Ausgangsmaterial durch Umfällen und Tiefgefrieren strukturell modifiziertes Chitosan eingesetzt. Mit einem solchen Ausgangsmaterial verläuft die Reaktion in besonders vorteilhafter Weise und besonders guter Ausbeute.

Anhand der nachfolgenden Beispiele wird das erfindungsgemässe Verfahren zur Herstellung der neuen quaternären Chitosanderivate näher erläutert.

Beispiel 1

Quaternisierung von Chitosan mit Glycidyltrimethylammoniumchlorid

100 g (0,62 mol) Chitosan mit einer Grenzviskositätszahl $\eta$=140 ml/g (ermittelt im DIN-Ubbelohde Viskosimeter mit 0,2 m Essigsäure und 0,1 m Natriumacetat als Lösungsmittel) und einem freien Amin-Gehalt von 86% wurden in einem Doppelmantelrührgefäss in 1 Liter Wasser mit 228 g Glycidyltrimethylammoniumchlorid umgesetzt. Die Zugabe des Glycidyltrimethylammoniumchlorids erfolgte in 3 Portionen zu jeweils 76 g im Abstand von 2,5 Stunden. Das Reaktionsgemisch wurde insgesamt 24 Stunden lang bei einer Temperatur von 60 °C gerührt. Danach wurde der Ansatz mit Salzsäure auf einen pH-Wert von 5,4 eingestellt und durch Gaze filtriert. Das Reaktionsprodukt wurde durch Eintropfen der wässrigen Lösung in Aceton ausgefällt, abgesaugt und schliesslich bei 50 °C im Vakuum getrocknet. Es wurden 115 g des quaternären Chitosanderivates erhalten.

Kenndaten des quaternären Chitosanderivates

| | |
|---|---|
| Grenzviskositätszahl: | $\eta$ = 27 ml/g |
| Titrierbarer Stickstoff: | 3,15 mmol/g |
| Chlorid-Bestimmung: | 13,17% (≙ 3,7 mmol/g) |
| Substitutionsgrad daraus berechnet: | 1,4 |

Die Wasserdampfaufnahme eines Filmes betrug bei 80% Luftfeuchte gegenüber 30% Luftfeuchte 22,45%. Die Pendelhärte nach König lag bei 17 sec.

Zur Berechnung des Substitutionsgrades wird zunächst die Molmenge an titrierbarem Stickstoff $N_t$[mmol/g] durch nichtwässrige Titration mit Perchlorsäure bestimmt. Das mittlere Molekulargewicht einer substituierten Chitosaneinheit berechnet sich daraus wie folgt:

$$\overline{M}\left[\frac{g}{mol}\right] = \frac{1000}{N_t\left[\frac{mmol}{g}\right]}$$

Weiterhin gilt die Gleichung

$$\overline{M} = 161 + x \cdot 152 + y \cdot 74 + z \cdot 42$$

- Molekulargewicht einer Chitosaneinheit = 161 [g/mol]
- Molekulargewicht einer quaternären Gruppe $R^3$ aus Formel I ($R^4$=$CH_3$,X=Cl) = 152 [g/mol]
- Molekulargewicht einer Glycidol-Gruppe $R^2$ aus Formel I = 74 [g/mol]
- Molekulargewicht einer $CO{-}CH_2$-Gruppe = 42 [g/mol]

x = Substitutionsgrad, kationische Gruppen $R^3$
y = Substitutionsgrad, Glycidol-Gruppen $R^2$
z = Substitutionsgrad des verwendeten Chitosans mit Acetyl-Gruppen (bei 60–96% freien Aminogruppen beträgt der Substitutionsgrad 0,04–0,4)

Zur Berechnung des Substitutionsgrades mit kationischen Gruppen $R^3$ wird die Chlorid-Titration mit der Ionensensitiven Chlorid-Elektrode angewendet. Es gilt

$$x = \frac{[Cl^-] \cdot M}{1000}$$

Daraus lässt sich der Substitutionsgrad mit Glycidol-Gruppen wie folgt berechnen:

$$y = \frac{\overline{M} - 161 - 152 \cdot x - 42 \cdot z}{74}$$

Beispiel 2

Umsetzung von Chitosan mit Glycidol und Glycidyltrimethylammoniumchlorid

100 g (0,62 mol) Chitosan (η = 140 ml/g; freies Amin 86%) wurden mit 79,5 g (1,07 mol) Glycidol und 80,6 g (0,53 mol) Glycidyltrimethylammoniumchlorid in 1 Liter Wasser bei 30 °C 48 Stunden lang unter Rühren umgesetzt. Anschliessend wurden weitere 26,3 g (0,36 mol) Glycidol und 25,8 g (0,17 mol) Glycidyltrimethylammoniumchlorid zugegeben und nochmals 24 Stunden lang bei 40 °C weitergerührt. Das alkalisch reagierende Reaktionsgemisch wurde mit Salzsäure auf einen pH-Wert von 5,5 eingestellt. Die erhaltene Lösung wurde 1 Woche lang dialysiert (Dialyseschlauch, Trenngrenze 1000) und das quaternäre Chitosanderivat durch Ausfällen in Aceton, Absaugen und Trocknen (bei 50 °C im Vakuum) gewonnen. Die Ausbeute des klar wasserlöslichen quaternären Chitosanderivates betrug 115 g.

Kenndaten des quaternären Chitosanderivates

| | |
|---|---|
| Grenzviskositätszahl: | η = 65 ml/g |
| Titrierbarer Stickstoff: | 3,07 mmol/g |
| Chlorid-Bestimmung: | 2,35% |
| | (≙ 0,69 mmol/g) |
| Substitutionsgrad: | |
| kationische Gruppen | 0,22 |
| Glycidol-Gruppen | 1,7 |
| Wasserdampfaufnahme: | 11,1% |
| Pendelhärte nach König: | 201 sec. |

Beispiel 3

50 g (0,31 mol) Chitosan mit den gleichen Kenndaten wie in Beispiel 2 wurden in einem Doppelmantelrührgefäss mit 31,5 ml (0,47 mol) Glycidol und 141,4 g (0,93 mol) Glycidyltrimethylammoniumchlorid sowie 500 ml Wasser 21 Stunden lang bei einer Temperatur von 80 °C gerührt. Anschliessend wurde das Reaktionsgemisch zentrifugiert und der Rückstand, bestehend aus nicht umgesetztem Chitosan, entfernt. Die wässrige Lösung wurde 1 Woche lang dialysiert und anschliessend, wie in Beispiel 2 beschrieben, aufgearbeitet. Die Ausbeute des quaternären Chitosanderivates betrug 35,7 g.

Kenndaten des quaternären Chitosanderivates

| | |
|---|---|
| Grenzviskositätszahl: | η = 41 ml/g |
| Titrierbarer Stickstoff: | 2,18 mmol/g |
| Chlorid-Bestimmung: | 8,88 |
| | (≙ 2,48 mmol/g) |
| Substitutionsgrad: | |
| kationische Gruppen | 1,10 |
| Glycidol-Gruppen | 1,6 |

Beispiel 4

Ein quaternäres Chitosan aus Beispiel 1 wird nachträglich mit Glycidol umgesetzt

20 g quaternäres Chitosan aus Beispiel 1 werden in 100 ml Wasser gelöst und bei 50 °C unter Rühren mit 50 ml (0,76 mol) Glycidol 48 Stunden lang zur Umsetzung gebracht. Anschliessend wurde das Reaktionsgemisch 1 Woche lang dialysiert und wie in Beispiel 2 beschrieben weiter aufgearbeitet. Die Ausbeute des quaternisierten Chitosanderivates betrug 8,8 g.

Kenndaten des quaternären Chitosanderivates

| | |
|---|---|
| Grenzviskositätszahl: | η = 51 ml/g |
| Titrierbarer Stickstoff: | 3,01 mmol/g |
| Chlorid-Bestimmung: | 6,4% |
| | (≙ 1,8 mmol/g) |
| Substitutionsgrad: | |
| kationische Gruppen | 0,6 |
| Glycidol-Gruppen | 1,0 |

Beispiel 5

Zunächst wird auf folgende Weise ein Glyceryl-Chitosan mit den Kenndaten η = 55 ml/g, Substitutionsgrad 1,7, hergestellt:

100 g Chitosan (η = 140 ml/g; freies Amin 86%) wurden in einem Doppelmantelrührgefäss mit 450 ml Glycidol (Molverhältnis 1:9) versetzt und das Gemisch 96 Stunden bei einer Temperatur von 80 °C gerührt. Das Reaktionsprodukt wurde in ca. 5 l Wasser gegeben, und der unlösliche Rückstand wurde abzentrifugiert. Die wässrige Lösung wurde eingeengt und das Reaktionsprodukt durch Ausfällen in Aceton isoliert. Nach dem Trocknen im Vakuum bei 50 °C wurde eine Ausbeute von 60 g erhalten.

50 g dieses Glyceryl-Chitosans wurden in 200 ml Wasser gelöst. Zu dieser Lösung wurden 5 g $Ba(OH)_2 \cdot 8\ H_2O$ und 23 g Glycidyltrimethylammoniumchlorid gegeben und das Gemisch 24 Stunden lang bei 50 °C gerührt. Die erhaltene wässrige Lösung wurde in Aceton eintropfen gelassen. Das ausgefallene quaternäre Chitosanderivat wurde abgesaugt und bei 50 °C im Vakuum getrocknet. Die Ausbeute betrug 37 g.

Kenndaten des quaternären Chitosanderivates

| | |
|---|---|
| Grenzviskositätszahl: | η = 35 ml/g |
| Titrierbarer Stickstoff: | 2,45 mmol/g |
| Chlorid-Bestimmung: | 1,5 mmol/g |
| Substitutionsgrad: | |
| kationische Gruppen | 0,6 |
| Glycidol-Gruppen | 2,0 |
| Wasserdampfaufnahme | 19,6% |
| Pendelhärte nach König: | 158 sec. |

Beispiel 6

Hochmolekulares Chitosan wird mit Salzsäure gelöst und mit Natronlauge wieder ausgefällt. Danach wird es mit Glycidol und Glycidyltrimethylammoniumchlorid umgesetzt.

15 g eines hochmolekularen Chitosans, mit den Kenndaten η = 1 600 ml/g und 76% freies Amin, wurden mit der äquimolaren Menge Salzsäure in 1 Liter Wasser gelöst und anschliessend durch

Einstellen eines pH-Wertes von 10 mit Natronlauge ausgefällt. Das ausgefällte und abgesaugte wasserhaltige Chitosan wurde mit 22 ml Glycidol sowie 24,2 g Glycidyltrimethylammoniumchlorid 15 Stunden lang bei 80 °C gerührt. Danach war das Chitosan völlig in Lösung gegangen. Diese wässrige Lösung wurde in Aceton eingetropft. Das ausgefallene kationische Chitosanderivat wurde abgesaugt und bei 50 °C im Vakuum getrocknet. Die Ausbeute des neuen Chitosanderivates betrug 22 g.

Kenndaten des quaternären Chitosanderivates

| | |
|---|---|
| Grenzviskositätszahl: | $\eta$ =733 ml/g |
| Titrierbarer Stickstoff: | 3,06 mmol/g |
| Chlorid-Bestimmung: | 1,56% (≙ 0,44 mmol/g) |
| Substitutionsgrad: | |
| kationische Gruppen | 0,14 |
| Glycidol-Gruppen | 1,3 |

Beispiel 7

50 g (0,31 mol) eines hochmolekularen Chitosans, mit den Kenndaten $\eta$ = 1 600 ml/g und 76% freies Amin, wurden mit der äquimolaren Menge Salzsäure in 5 Liter Wasser gelöst und anschliessend durch Einstellen eines pH-Wertes von 10 mit Natronlauge ausgefällt. Das ausgefällte und abgesaugte wasserhaltige Chitosan wurde in einem Doppelmantelrührgefäss von 2 Liter Inhalt mit 73,3 ml (1,1 mol) Glycidol sowie 80,6 g Glycidyltrimethylammoniumchlorid 8 Stunden lang bei 80 °C gerührt. Danach wurde wie in Beispiel 6 beschrieben weiterverfahren. Die Ausbeute des Chitosanderivates betrug 85 g.

Kenndaten des quaternären Chitosanderivates

| | |
|---|---|
| Grenzviskositätszahl: | $\eta$ =710 ml/g |
| Titrierbarer Stickstoff: | 3,2 mmol/g |
| Chlorid-Bestimmung: | 1,80 mmol/g |
| Substitutionsgrad: | |
| kationische Gruppen | 0,60 |
| Glycidol-Gruppen | 2,1 |
| Wasserdampfaufnahme: | 12,2% |
| Pendelhärte nach König: | 193 sec. |

Beispiel 8

Quaternisierung von Chitosan mit 1-Chlor-3-trimethylammonium- propanol-2-chlorid

10 g (0,062 mol) Chitosan ($\eta$ = 140 ml/g; freies Amin 86%) wurden mit 58 g (0,18 mol) 60%igem 1-Chlor-3-trimethylammonium- propanol-2-chlorid und 10 g (0,25 mol) Natriumhydroxid in 100 ml Wasser 72 Stunden lang bei 50 °C gerührt. Anschliessend wurde das unlösliche Reaktionsprodukt abgesaugt, mit Wasser gewaschen und sodann mit 15 ml Glycidol 48 Stunden lang bei 50 °C gerührt. Der danach noch verbliebene wasserunlösliche Rückstand, bestehend aus nicht umgesetztem Chitosan, wurde durch Zentrifugieren abgetrennt. Die wässrige Lösung wurde dekantiert und 1 Woche lang dialysiert. Anschliessend wurde die Lösung wie in Beispiel 2 beschrieben weiter verarbeitet. Die Ausbeute an dem quaternären Chitosanderivat betrug 10,8 g.

Kenndaten des quaternären Chitosanderivates

| | |
|---|---|
| Grenzviskositätszahl: | $\eta$ =57 ml/g |
| Titrierbarer Stickstoff: | 3,15 mmol/g |
| Chlorid-Bestimmung: | 0,5 (≙ 0,15 mmol/g) |
| Substitutionsgrad: | |
| kationische Gruppen | 0,05 |
| Glycidol-Gruppen | 1,9 |
| Wasserdampfaufnahme: | 21,1% |
| Pendelhärte nach König: | 196 sec. |

Beispiel 9

100 g (0,62 mol) Chitosan mit den gleichen Kenndaten wie in Beispiel 2 wurden in 400 ml Wasser dispergiert. Dieser Dispersion wurden bei einer Temperatur von 80 °C unter Rühren in Abständen von 2,5 Stunden 3-mal je 70 g (0,5 mol) Glycidyltrimethylammoniumchlorid zugegeben.

Nach einer Reaktionszeit von 24 Stunden wurden dem Reaktionsgemisch 250 ml (3,9 mol) Glycidol zugegeben, und das Gemisch wurde erneut 24 Stunden lang bei 80 °C gerührt. Das Reaktionsgemisch wurde zentrifugiert. Die klare Lösung des Reaktionsproduktes wurde 1 Woche lang dialysiert und sodann wie in Beispiel 2 beschrieben weiterverarbeitet. Die Ausbeute an dem quaternären Chitosanderivat betrug 66,7 g.

Kenndaten des quaternären Chitosanderivates

| | |
|---|---|
| Grenzviskositätszahl: | $\eta$ =43 mol/g |
| Titrierbarer Stickstoff: | 2,64 mmol/g |
| Chlorid-Bestimmung: | 2,05 mmol/g |
| Substitutionsgrad: | |
| kationische Gruppen | 0,8 |
| Glycidol-Gruppen | 1,2 |

Nachfolgend werden Beispiele für kosmetische Mittel auf der Basis der neuen erfindungsgemässen quaternären Chitosanderivate gegeben:

Beispiel 10

Haarfestiger

| | |
|---|---|
| 0,6 g | quaternäres Chitosanderivat nach Beispiel 2 ($\eta$ =65 ml/g, Substitutionsgrad = kationische Gruppen 0,22; Glycidol-Gruppen 1,7) |
| 73,8 g | Wasser |
| 25,0 g | Isopropanol |
| 0,4 g | 10%ige Ameisensäure |
| 0,2 g | Parfümöl |
| 100,0 g | |

20 ml dieser Lösung wurden auf dem gewaschenen, handtuchtrockenen Haar verteilt, das Haar in üblicher Weise zur Frisur eingelegt und getrocknet. Bei guter Festigungswirkung zeigte das Haar, im Vergleich zu einem Haarfestiger auf der Basis von Chitosan/Ameisensäure, einen angenehmeren und weicheren Griff.

Beispiel 11

Tönungsfestiger

| | |
|---|---|
| 1,00 g | quaternäres Chitosanderivat nach Beispiel 7 (η = 710 ml/g, Substitutionsgrad = kationische Gruppen 0,60; Glycidol-Gruppen 2,1) |
| 1,00 g | Milchsäure |
| 0,10 g | Cetyltrimethylammoniumchlorid, 50%ige wässrige Lösung |
| 0,05 g | Acid Brown 4 (C.I. 41 805) |
| 97,85 g | Wasser |
| 100,00 g | |

20 ml dieser Lösung wurden auf dem gewaschenen, handtuchtrockenen Haar verteilt und das Haar in üblicher Weise eingelegt und getrocknet. Das Haar zeigte anschliessend eine leichte Rot-Braunfärbung.

Beispiel 12

Tönungsfestiger

| | |
|---|---|
| 0,60 g | quaternäres Chitosanderivat nach Beispiel 2 (η = 65 ml/g, Substitutionsgrad = kationische Gruppen 0,22; Glycidol-Gruppen 1,7) |
| 0,15 g | 1,4- Di(β-hydroxyethylamino)-2-nitro-5-chlorbenzol |
| 25,00 g | Ethanol |
| 74,25 g | Wasser |
| 100,00 g | |

20 ml dieser Lösung wurden auf die gewaschenen, handtuchtrockenen Haare gegeben, sodann das Haar eingelegt und getrocknet. Die Haare waren rot-violett gefärbt und gefestigt.

Beispiel 13

Anionisches Haarwaschmittel

| | |
|---|---|
| 1,00 g | quaternäres Chitosanderivat nach Beispiel 4 (η = 51 ml/g, Substitutionsgrad = kationische Gruppen 0,6; Glycidol-Gruppen 1,0) |
| 40,00 g | Laurylalkoholdiglykolethersulfat-Natriumsalz, 28%ige wässrige Lösung |
| 4,00 g | Natriumchlorid |
| 0,05 g | Farbstoff |
| 54,85 g | Wasser |
| 0,10 g | Formaldehyd, 25%ige wässrige Lösung |
| 100,00 g | |

Es wurde ein klares Shampoo erhalten. Das damit gewaschene Haar war hinsichtlich Griff, Glanz und Kämmbarkeit ausgezeichnet konditioniert. Infolge der Verträglichkeit des quaternären Chitosanderivates mit Alkylethersulfat kann das vorstehend aufgeführte Shampoo erstellt werden, mit dem eine gleichzeitige Reinigung und Pflege des Haares möglich ist.

Beispiel 14

Amphoteres, tönendes Haarwaschmittel

| | |
|---|---|
| 2,00 g | quaternäres Chitosanderivat nach Beispiel 4 (η = 51 ml/g, Substitutionsgrad = kationische Gruppen 0,6; Glycidol-Gruppen 1,0) |
| 40,00 g | Dimethyl-carboxymethylen-propylenamidostearatbetain, 35%ige wässrige Lösung |
| 5,06 g | Ameisensäure, 10%ig |
| 3,50 g | Kokosfettsäurediethanolamid |
| 1,00 g | Pikraminsäure (C.I. 76 540), 1%ige wässrige Lösung) |
| 48,44 g | Wasser, vollentsalzt |
| 100,00 g | |

Mit etwa 15 bis 20 g des obigen Mittels wurde das Haar einshampooniert. Nach einer Einwirkungszeit von 5 bis 10 Minuten spülte man mit Wasser aus. Das Haar war gelb-orange getönt und ausgezeichnet konditioniert, besonders hinsichtlich Griff und Kämmbarkeit.

Beispiel 15

Haarkurmittel, kationisch

| | |
|---|---|
| 0,30 g | quaternäres Chitosanderivat nach Beispiel 7 (η = 710 ml/g, Substitutionsgrad = kationische Gruppen 0,60; Glycidol-Gruppen 2,1) |
| 4,00 g | Cetylstearylalkohol |
| 1,48 g | Milchsäure, 10%ig |
| 2,50 g | Kokos(pentaethoxy)methylammoniumchlorid |
| 1,00 g | Sorbitanmonopalmitat mit 20 Mol Äthylenoxid |
| 90,72 g | Wasser, vollentsalzt |
| 100,00 g | |

Beispiel 16

Haarkurmittel, gelförmig

| | |
|---|---|
| 2,1 g | quaternäres Chitosanderivat nach Beispiel 4 (η = 51 ml/g, Substitutionsgrad = kationische Gruppen 0,6; Glycidol-Gruppen 1,0) |
| 0,6 g | Hydroxypropylmethylcellulose |
| 0,5 g | Laurylpyridiniumchlorid |
| 96,8 g | Wasser, vollentsalzt |
| 100,0 g | (auf pH 5,0 mit 10%iger Ameisensäure eingestellt) |

Jeweils 35 g der Haarkurmittel nach Beispiel 15 bzw. 16 wurden im gewaschenen Haar verteilt und nach einer Einwirkungszeit von 3 bis 5 Minuten mit Wasser wieder ausgespült; als Ergebnis wurde ein ausgezeichneter Griff, Glanz sowie Kämmbarkeit des Haares erhalten.

Beispiel 17

Hautcreme

| | |
|---|---|
| 0,30 g | quaternäres Chitosanderivat nach Beispiel 2 (η = 65 ml/g, Substitutionsgrad = kationische Gruppen 0,22; Glycidol-Gruppen 1,7) |
| 3,00 g | Stearylalkohol |
| 1,00 g | Wollfettalkohol (adeps lanae) |
| 1,00 g | Vaseline |
| 0,76 g | Milchsäure, 10%ig |
| 1,00 g | Natriumcetylstearylsulfat |
| 92,94 g | Wasser, vollentsalzt |
| 100,00 g | |

Beispiel 18
Haartönungsmittel

| | |
|---|---|
| 0,50 g | quaternäres Chitosanderivat nach Beispiel 7 ($\eta$ = 710 ml/g, Substitutionsgrad = kationische Gruppen 0,60; Glycidol-Gruppen 2,1) |
| 12,00 g | Cetylstearylalkohol |
| 0,10 g | Parahydroxybenzoesäureethylester |
| 6,00 g | Laurylalkohol-digylcolethersulfat-Natriumsalz (28%ige wässrige Lösung) |
| 0,50 g | Parfümöl |
| 79,31 g | Wasser |
| 0,50 g | 1-Hydroxy-2-amino-4-nitrobenzol (C.I. 76 530) |
| 0,85 g | 1,4-Diamino-2-nitrobenzol (C.I. 76 070) |
| 0,24 g | Natriumhydroxyd |
| 100,00 g | |

Ungefähr 30 bis 40 g wurden in dem gewaschenen Haar verteilt und nach einer Einwirkungszeit von 20 Minuten ausgespült. Das Haar war rötlich gefärbt und wies eine gute Kämmbarkeit und einen angenehmen Griff auf.

Beispiel 19
Oxidationshaarfärbemittel

| | |
|---|---|
| 0,50 g | quaternäres Chitosanderivat nach Beispiel 7 ($\eta$ = 710 ml/g, Substitutionsgrad = kationische Gruppen 0,60; Glycidol-Gruppen 2,1) |
| 0,08 g | 3,5-Diamino-2,6-dimethoxypyridin-dihydrochlorid |
| 0,30 g | 1,4-Diaminobenzol |
| 0,25 g | Resorcin |
| 0,30 g | Natriumsulfit |
| 3,50 g | Laurylalkohol-diglycolethersulfat-Natriumsalz (28%ige wässrige Lösung) |
| 15,00 g | Cetylakohol |
| 3,00 g | Ammoniak |
| 77,07 g | Wasser |
| 100,00 g | |

50 g dieses Haarfärbemittels wurden mit 50 ml 6%iger Wasserstoffperoxidlösung gemischt und auf weisses Haar aufgetragen. Nach 30 Minuten wurde das Haar mit Wasser ausgespült und getrocknet. Das Haar hatte eine natürlich wirkende matt-blonde Färbung sowie einen natürlichen angenehmen Griff.

Beispiel 20
Dauerwellmittel

| | |
|---|---|
| 0,5 g | quaternäres Chitosanderivat nach Beispiel 7 ($\eta$ = 710 ml/g, Substitutionsgrad = kationische Gruppen 0,60; Glycidol-Gruppen 2,1) |
| 10,0 g | Thioglykolsäure |
| 8,0 g | Ammoniak, 25%ig |
| 6,1 g | Ammoniumhydrogencarbonat |
| 75,4 g | Wasser |
| 100,0 g | |

Zur Anwendung trug man dieses Dauerwellmittel auf das gewickelte handtuchtrockene Haar gleichmässig auf und liess es etwa 20 Minuten einwirken; danach wurde das Haar mit Wasser ausgespült und in bekannter Weise oxidativ behandelt. Es wurde ein gutes Wellergebnis erhalten, die Haare fühlten sich natürlich und weich an.

Beispiel 21
Haarfestiger Alkoholfrei

| | |
|---|---|
| 0,7 g | quaternäres Chitosanderivat nach Beispiel 8 ($\eta$ = 57 ml/g, Substitutionsgrad = kationische Gruppen 0,05; Glycidol-Gruppen 1,9) |
| 1,5 g | Ameisensäure 10%ig |
| 0,8 g | Parfüm |
| 0,1 g | Chloracetamid (Konservierungsmittel) |
| 96,9 g | Wasser, vollentsalzt |
| 100,0 g | |

20 ml dieser Lösung wurden auf die gewaschenen handtuchtrockenen Haare gegeben, sodann das Haar eingelegt und getrocknet. Bei guter Festigungswirkung zeigte das Haar einen angenehmen weichen Griff.

## Patentansprüche

1. Kosmetisches Mittel zur Behandlung von Haaren oder der Haut, dadurch gekennzeichnet, dass es in einer geeigneten Kosmetikgrundlage eine quaternäre makromolekulare, vom Chitosan abgeleitete polymere Verbindung der allgemeinen Formel I

$$HO\left[\,C_6H_{11-m}NO_4(R^1)_m(R^2)_n(R^3)_q\,\right]_p H, \qquad (I)$$

wobei m jeden beliebigen Zahlenwert von 0 bis 0,5 bedeutet, n jeden beliebigen Zahlenwert von 0 bis 6 bedeutet, q ein beliebiger Zahlenwert von 0,005 bis 3,0 ist, p eine ganze Zahl von 10 bis 50 000 bedeutet, $R^1$ ein Acetyl-Rest ist, $R^2$ einen zweiwertigen Rest $-CH_2CH(OH)-CH_2-O-$,

$-CH_2-CH(-CH_2-OH)-O-$ oder $-CH(-CH_2-O-)-CH_2-OH$

darstellt und $R^3$ ein zweiwertiger Rest

$-CH_2-CH(-CH_2-N^+(R^4)_3\,X^-)-O-$ oder $-CH(-CH_2-N^+(R^4)_3\,X^-)-CH_2-O-$

mit $R^4$ = $C_1$- bis $C_4$-Alkyl und X = Cl, Br, J oder $CH_3SO_4$ ist, enthält.

2. Mittel nach Anspruch 1, dadurch gekennzeichnet, dass n in der Formel I einen Zahlenwert von 1,0 bis 2,1 bedeutet.

3. Mittel nach Anspruch 1 und 2, dadurch gekennzeichnet, dass q in der Formel I einen Zahlenwert von 0,05 bis 0,6 bedeutet.

4. Mittel nach Anspruch 1 bis 3, dadurch gekennzeichnet, dass es die polymere Verbindung

der Formel I in einer Menge von 0,05 bis 10,0 Gew.-% enthält.

5. Mittel nach Anspruch 1 bis 4, dadurch gekennzeichnet, dass es als Kosmetikgrundlage eine wässrige, alkoholische oder wässrig-alkoholische Lösung, eine Creme, ein Gel oder eine Emulsion enthält.

6. Mittel nach Anspruch 1 bis 5, dadurch gekennzeichnet, dass es als Kosmetikgrundlage eine wässrige oder wässrig-alkoholische Lösung eines niedermolekularen Alkohols, wie Ethanol oder Isopropanol, umfasst und einen pH-Wert zwischen 6 und 8 aufweist.

7. Mittel nach Anspruch 1 bis 5, dadurch gekennzeichnet, dass es zusätzlich ein kationisches, nicht-ionisches, amphoteres oder anionisches Tensid enthält und in Form eines Shampoos vorliegt.

8. Mittel nach Anspruch 7, dadurch gekennzeichnet, dass das anionische Tensid ein Alkylethersulfat ist.

9. Mittel nach Anspruch 7 und 8, dadurch gekennzeichnet, dass es das Tensid in einer Konzentration zwischen 3 und 25 Gew.-% enthält und einen pH-Wert zwischen 4 und 7 aufweist.

10. Mittel nach Anspruch 1 bis 6, dadurch gekennzeichnet, dass es als Kosmetikgrundlage eine alkoholische oder wässrig-alkoholische Lösung enthält, die mit einem unter Druck verflüssigten Treibgas vermischt ist, in einem Druckbehälter abgefüllt ist und in Form eines Aerosolhaarsprays vorliegt.

11. Mittel nach Anspruch 1 bis 6, dadurch gekennzeichnet, dass die Kosmetikgrundlage eine wässrige, alkoholische oder wässrig-alkoholische Lösung, eine Creme, ein Gel oder eine Emulsion ist, in der das Chitosanderivat der Formel I in einer Konzentration zwischen 0,1 und 10 Gew.-% enthalten ist und es in Form eines Hautbehandlungsmittels vorliegt.

12. Mittel nach Anspruch 1 bis 10, dadurch gekennzeichnet, dass es zusätzlich ein bekanntes filmbildendes synthetisches oder natürliches kosmetisches Polymer enthält.

13. Mittel nach Anspruch 1 bis 5, dadurch gekennzeichnet, dass die Kosmetikgrundlage eine wässrige, alkoholische oder wässrig-alkoholische Lösung oder eine Emulsion ist, welche zusätzlich einen Farbstoff enthält und in Form eines Farbfestigers oder Tönungsfestigers vorliegt.

14. Quaternäre makromolekulare, vom Chitosan abgeleitete polymere Verbindung der allgemeinen Formel I

$$HO\left[\ C_6H_{11-m}NO_4(R^1)_m(R^2)_n(R^3)_q\ \right]_p H, \qquad (I)$$

wobei m jeden beliebigen Zahlenwert von 0 bis 0,5 bedeutet, n jeden beliebigen Zahlenwert von 0 bis 6 bedeutet, q ein beliebiger Zahlenwert von 0,005 bis 3,0 ist, p eine ganze Zahl von 10 bis 50 000 bedeutet, $R^1$ ein Acetyl-Rest ist, $R^2$ einen zweiwertigen Rest $-CH_2CH(OH)-CH_2-O-$,

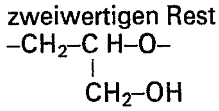
oder
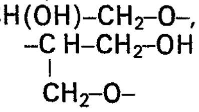

darstellt und $R^3$ ein zweiwertiger Rest

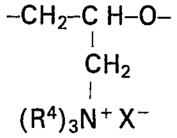
oder
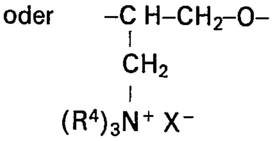

mit $R^4 = C_1$- bis $C_4$-Alkyl und X = Cl, Br, J oder $CH_3SO_4$ ist.

15. Verbindung gemäss Anspruch 14, dadurch gekennzeichnet, dass in der Formel I n einen Zahlenwert von 1,0 bis 2,1 bedeutet, q einen Zahlenwert von 0,05 bis 1,1 darstellt und die Grenzviskositätszahl η einen Wert von 27 bis 733 ml/g aufweist.

16. Verbindung gemäss Anspruch 14, dadurch gekennzeichnet, dass in der Formel I n = 0 bedeutet und q = 1,4 ist.

17. Verbindung gemäss Anspruch 14, dadurch gekennzeichnet, dass in der Formel I $R^4 = CH_3$ und X = Cl bedeuten.

18. Verfahren zur Herstellung der Verbindungen nach Anspruch 14, dadurch gekennzeichnet, dass man ein Chitosan, bestehend aus zu 50–100% entacetyliertem Chitin, mit einem Glycidyltrialkylammoniumhalogenid sowie gegebenenfalls zusätzlich mit Glycidol (1,2-Epoxypropanol-3) in geeignetem Verhältnis gleichzeitig oder in beliebiger Reihenfolge zur Umsetzung bringt.

19. Verfahren nach Anspruch 18, dadurch gekennzeichnet, dass man das Chitosan in Gegenwart eines Lösungsmittels mit dem Glycidyltrialkylammoniumhalogenid und gegebenenfalls dem Glycidol vermischt und diese Mischung bei einer Temperatur zwischen 10 und 100 °C mehrere Stunden mischt oder rührt.

20. Verfahren nach Anspruch 18 und 19, dadurch gekennzeichnet, dass die Umsetzung in Gegenwart einer organischen oder anorganischen Base durchgeführt wird.

21. Verfahren nach Anspruch 18 und 19, dadurch gekennzeichnet, dass die Reaktion in Gegenwart einer organischen oder anorganischen Säure durchgeführt wird.

22. Verfahren nach Anspruch 18 bis 21, dadurch gekennzeichnet, dass man als Glycidyltrialkylammoniumhalogenid Glycidyltrimethylammoniumchlorid verwendet.

23. Verfahren nach Anspruch 18 bis 22, dadurch gekennzeichnet, dass man als Ausgangsmaterial durch Umfüllen und Tiefgefrieren strukturell modifiziertes Chitosan verwendet.

24. Verfahren nach Anspruch 18 bis 23, dadurch gekennzeichnet, dass man das Chitosan in einer ersten Stufe mit Glycidol und in einer zweiten Stufe mit einem Glycidyltrialkylammoniumhalogenid umsetzt.

25. Verfahren nach Anspruch 18 bis 24, dadurch gekennzeichnet, dass man die Umsetzung in Gegenwart von Wasser durchführt.

## Revendications

1. Agent cosmétique pour le traitement des cheveux ou de la peau, caractérisé en ce qu'il contient dans une base cosmétique appropriée, une combinaison polymère macromoléculaire quaternaire dérivée du chitosane, de formule générale I

$$HO\left[\ C_6H_{11-m}NO_4(R^1)_m(R^2)_n(R^3)_q\ \right]_p H, \qquad (I)$$

où m représente une valeur numérique quelconque comprise entre 0 et 0,5, n une valeur numérique quelconque comprise entre 0 et 6, q une valeur numérique quelconque comprise entre 0,005 et 3,0, p un nombre entier compris entre 10 et 50 000, $R^1$ un radical acétyle, $R^2$ un reste bivalent

$-CH_2CH(OH)-CH_2-O-$,

$$-CH_2-\underset{\displaystyle CH_2-OH}{\underset{|}{C}}H-O- \quad \text{ou} \quad -\underset{\displaystyle CH_2-O-}{\underset{|}{C}}H-CH_2-OH$$

et $R^3$ un reste bivalent

$$-CH_2-\underset{\displaystyle (R^4)_3N^+X^-}{\underset{|}{\underset{\displaystyle CH_2}{\underset{|}{C}}}}H-O- \quad \text{ou} \quad -\underset{\displaystyle (R^4)_3N^+X^-}{\underset{|}{\underset{\displaystyle CH_2}{\underset{|}{C}}}}H-CH_2-O-$$

avec $R^4$ = $C_1$- à $C_4$-alkyle et X = Cl, Br, J ou $CH_3SO_4$.

2. Agent selon la revendication 1, caractérisé en ce que n de la formule I désigne une valeur numérique comprise entre 1,0 et 2,1.

3. Agent selon la revendication 1 ou 2, caractérisé en ce que q de la formule I désigne une valeur numérique comprise entre 0,05 et 0,6.

4. Agent selon l'une quelconque des revendications 1 à 3, caractérisé en ce qu'il contient la combinaison polymère de la formule I selon une quantité comprise entre 0,05 et 10,0% en poids.

5. Agent selon l'une quelconque des revendications 1 à 4, caractérisé en ce qu'il contient en tant que base cosmétique une solution aqueuse, alcoolique ou alcoolique-aqueuse, une crème, un gel ou une émulsion.

6. Agent selon l'une quelconque des revendications 1 à 5, caractérisé en ce qu'il contient en tant que base cosmétique une solution aqueuse, alcoolique ou alcoolique-aqueuse d'un alcool de faible poids moléculaire tel que l'éthanol ou l'isopropanol, et présente une valeur de pH comprise entre 6 et 8.

7. Agent selon l'une quelconque des revendications 1 à 5, caractérisé en ce qu'il contient en plus un agent tensio-actif non ionique, amphotère, cationique ou anionique, et se présente sous la forme d'un shampooing.

8. Agent selon la revendication 7, caractérisé en ce que l'agent tensio-actif anionique est un sulfate d'alkyl-éther.

9. Agent selon la revendication 7 ou 8, caractérisé en ce qu'il contient l'agent tensio-actif selon une concentration comprise entre 3 et 25% en poids et présente une valeur de pH comprise entre 4 et 7.

10. Agent selon l'une quelconque des revendications 1 à 6, caractérisé en ce qu'il contient en tant que base cosmétique une solution alcoolique ou alcoolique-aqueuse qui est mélangée avec un gaz propulseur liquéfié sous pression, est mis dans un récipient à pression et se présente sous la forme d'un spray d'aérosol pour cheveux.

11. Agent selon l'une quelconque des revendications 1 à 6, caractérisé en ce que la base cosmétique est une solution aqueuse, alcoolique ou alcoolique-aqueuse, une crème, un gel ou une émulsion, dans laquelle est contenu le dérivé de chitosane de la formule I selon une concentration comprise entre 0,1 et 10% en poids et se présente sous la forme d'une agent de traitement de la peau.

12. Agent selon l'une quelconque des revendications 1 à 10, caractérisé en ce qu'il contient en plus un polymère cosmétique synthétique ou naturel connu et formateur de film.

13. Agent selon l'une quelconque des revendications 1 à 5, caractérisé en ce que la base cosmétique est constituée par une solution ou une émulsion aqueuse, alcoolique ou alcoolique-aqueuse, qui contient en plus un colorant et se présente sous forme d'un renforçateur colorant ou d'un renforçateur nuanceur.

14. Combinaison polymère macromoléculaire quaternaire dérivée du chitosane, de formule générale I

$$HO\left[\ C_6H_{11-m}NO_4(R^1)_m(R^2)_n(R^3)_q\ \right]_p H, \qquad (I)$$

où m représente une valeur numérique quelconque comprise entre 0 et 0,5, n une valeur numérique quelconque comprise entre 0 et 6, q une valeur numérique quelconque comprise entre 0,005 et 3,0, p un nombre entier compris entre 10 et 50 000, $R^1$ un radical acétyle, $R^2$ un reste bivalent

$-CH_2CH(OH)-CH_2-O-$,

$$-CH_2-\underset{\displaystyle CH_2-OH}{\underset{|}{C}}H-O- \quad \text{ou} \quad -\underset{\displaystyle CH_2-O-}{\underset{|}{C}}H-CH_2-OH$$

et $R^3$ un reste bivalent

$$-CH_2-\underset{\displaystyle (R^4)_3N^+X^-}{\underset{|}{\underset{\displaystyle CH_2}{\underset{|}{C}}}}H-O- \quad \text{ou} \quad -\underset{\displaystyle (R^4)_3N^+X^-}{\underset{|}{\underset{\displaystyle CH_2}{\underset{|}{C}}}}H-CH_2-O-$$

avec $R^4$ = $C_1$- à $C_4$-alkyle et X = Cl, Br, J ou $CH_3SO_4$.

15. Combinaison selon la revendication 14, caractérisée en ce que dans la formule I, n représente une valeur numérique comprise entre 1,0 et 2,1, q représente une valeur numérique comprise entre 0,05 et 1,1 et en ce que le nombre limite η de la viscosité a une valeur comprise entre 27 et 733 ml/g.

16. Combinaison selon la revendication 14, caractérisée en ce que dans la formule I, n = 0 et q = 1,4.

17. Combinaison selon la revendication 14, caractérisée en ce que dans la formule I, $R^4 = CH_3$ et X = Cl.

18. Procédé de préparation des combinaisons selon la revendication 14, caractérisé en ce que l'on fait réagir un chitosane constitué par de la chitine désacétylée à 50–100%, avec un halogénure de glycidyltrialkylammonium ainsi qu'éventuellement et en plus avec du glycidol (1,2-époxy-propanol-3) selon un rapport approprié, simultanément ou selon une séquence quelconque.

19. Procédé selon la revendication 18, caractérisé en ce que l'on mélange le chitosane en présence d'un solvant avec l'halogénure de glycidyltrialkylammonium et éventuellement le glycidol, et en ce que l'on mélange ou l'on brasse ce mélange pendant plusieurs heures à une température comprise entre 10 °C et 100 °C.

20. Procédé selon la revendication 18 ou 19, caractérisé en ce que la réaction est réalisée en présence d'une base organique ou inorganique.

21. Procédé selon la revendication 18 ou 19, caractérisé en ce que la réaction est réalisée en présence d'un acide organique ou inorganique.

22. Procédé selon l'une quelconque des revendications 18 à 21, caractérisé en ce que l'on utilise en tant qu'halogénure de glycidyltrialkylammonium du chlorure de glycidyltrialkylammonium.

23. Procédé selon l'une quelconque des revendications 18 à 22, caractérisé en ce que l'on utilise en tant que produit de départ du chitosane structurellement modifié par transvasement et surgélation.

24. Procédé selon l'une quelconque des revendications 18 à 23, caractérisé en ce que l'on fait réagir le chitosane avec du glycidol au cours d'une première étape et avec de l'halogénure deglycidyltrialkylammonium au cours d'une seconde étape.

25. Procédé selon l'une quelconque des revendications 18 à 24, caractérisé en ce que l'on réalise la réaction en présence d'eau.

## Claims

1. Cosmetic composition for the treatment of hair or the skin, characterised in that it contains, in a suitable cosmetic base, a quaternary macro-molecular polymeric compound derived from Chitosan of the general Formula I

$$HO\left[C_6H_{11-m}NO_4(R^1)_m(R^2)_n(R^3)_q\right]_pH, \qquad (I)$$

in which m denotes any desired numerical value from 0 to 0.5, n any desired numerical value from 0 to 6, q is any desired numerical value from 0.005 to 3.0, p is a whole number from 10 to 50 000, $R^1$ is an acetyl radical, $R^2$ is a bivalent radical

$-CH_2CH(OH)-CH_2-O-$,

$-CH_2-CH-O-$ (with $CH_2-OH$ on the CH) or $-CH-CH_2-OH$ (with $CH_2-O-$ on the CH)

and in which $R^3$ is a bivalent radical

$-CH_2-CH-O-$ (with $CH_2$–$(R^4)_3N^+X^-$ on the CH) or $-CH-CH_2-O-$ (with $CH_2$–$(R^4)_3N^+X^-$ on the CH)

in which $R^4$ = $C_1$- to $C_4$-alkyl and X = Cl, Br, I or $CH_3SO_4$.

2. Composition according to Claim 1, characterised in that n in Formula I denotes a numerical value from 1.0 to 2.1.

3. Composition according to Claim 1 and 2, characterised in that in Formula I, q denotes a numerical value from 0.05 to 0.6.

4. Composition according to Claim 1 to 3, characterised in that it contains the polymeric compound of Formula I in a quantity from 0.05 to 10.0% by weight.

5. Composition according to Claim 1 to 4, characterised in that as a cosmetic base it contains an aqueous, alcoholic or aqueous-alcoholic solution, a cream, a gel or an emulsion.

6. Composition according to Claim 1 to 5, characterised in that as a cosmetic base it contains an aqueous or aqueous-alcoholic solution of a low-molecular alcohol, such as ethanol or isopropanol and has a pH value between 6 and 8.

7. Composition according to Claim 1 to 5, characterised in that it additionally contains a cationic, non-ionic, amphoteric or anionic tenside and takes the form of a shampoo.

8. Composition according to Claim 7, characterised in that the anionic tenside is an alkylethersulphate.

9. Composition according to Claims 7 and 8, characterised in that it contains the tenside in a concentration between 3 and 25% by weight and has a pH value between 4 and 7.

10. Composition according to Claim 1 to 6, characterised in that as a cosmetic base it contains an alcoholic or aqueous-alcoholic solution blended with a pressurised liquefied propellent gas, is filled into a pressure-resistant container and presented in the form of an aerosol hairspray.

11. Composition according to Claim 1 to 6, characterised in that the cosmetic base is an aqueous, alcoholic or aqueous-alcoholic solution, a cream, a gel, or an emulsion in which the Chitosan derivative of Formula I is contained in a concentration of between 0.1 and 10% by weight and takes the form of a skin treatment agent.

12. Composition according to Claim 1 to 10, characterised in that in addition it contains a

known film-forming synthetic or natural cosmetic polymer.

13. Composition according to Claim 1 to 5, characterised in that the cosmetic base is an aqueous, alcoholic or aqueous-alcoholic solution or an emulsion which additionally contains a dyestuff and which is present in the form of a colouring or toning setting lotion.

14. Quaternary macro-molecular polymeric compound derived from Chitosan of the general Formula I

$$HO\left[\ C_6H_{11-m}NO_4(R^1)_m(R^2)_n(R^3)_q\ \right]_p H, \qquad (I)$$

in which m denotes any desired numerical value from 0 to 0.5, n any desired numerical value from 0 to 6, q is any desired numerical value from 0.005 to 3.0, p is a whole number from 10 to 50 000, $R^1$ is an acetyl radical, $R^2$ is a bivalent radical

$-CH_2CH(OH)-CH_2-O-$,

```
-CH2-C H-O-        or        -C H-CH2-OH
     |                          |
     CH2-OH                     CH2-O-
```

and $R^3$ is a bivalent radical

```
-CH2-C H-O-        or        -C H-CH2-O-
     |                          |
     CH2                        CH2
     |                          |
 (R4)3N+ X-                 (R4)3N+ X-
```

with $R^4$ = $C_1$- to $C_4$-alkyl and X = Cl, Br, I or $CH_3SO_4$.

15. Compound according to Claim 14, characterised in that in Formula I, n denotes a numerical value from 1.0 to 2.1, q a numerical value from 0.05 to 1.1 and the limit viscosity number η has a value of 27 to 733 ml/g.

16. Compound according to Claim 14, characterised in that in Formula I, n = 0 and q = 1.4.

17. Compound according to Claim 14, characterised in that in Formula I $R^4$ = CH and X = Cl.

18. Method of producing the compounds according to Claim 14, characterised in that a Chitosan consisting of 50–100% deacetylated Chitin is caused to react in a suitable proportion simultaneously or in any desired sequence with a glycidyltrialkylammoniumhalide and possibly also with glycidol (1.2-epoxy-propanol-3).

19. Method according to Claim 18, characterised in that the Chitosan is, in the presence of a solvent, blended with the glycidyltrialkylammoniumhalide and possibly the glycidol, this mixture being blended or agitated for several hours at a temperature of between 10 and 100 degrees C.

20. Method according to Claim 18 and 19, characterised in that the reaction is carried out in the presence of an organic or inorganic base.

21. Method according to Claim 18 and 19, characterised in that the reaction is carried out in the presence of an organic or inorganic acid.

22. Method according to Claim 18 to 21, characterised in that glycidyltrialkylammoniumchloride is used as the glycidyltrialkylammoniumhalide.

23. Method according to Claim 18 to 22, characterised in that the starting material used is Chitosan which has been structurally modified by transfer and deep-freezing.

24. Method according to Claim 18 to 23, characterised in that in a first stage the Chitosan is reacted with glycidol and in the second stage with a glycidyltrialkylammoniumhalide.

25. Method according to Claim 18 to 24, characterised in that the reaction is carried out in the presence of water.